# FASCICULE DE BREVET EUROPEEN

(11) **EP 2 555 740 B1**
(45) Date de publication et mention de la délivrance du brevet: **27.07.2016**
(21) Numéro de dépôt: 11718456.4
(22) Date de dépôt: 05.04.2011
(51) Int. Cl.: A61K 6/00, A61K 6/02, A61L 26/00, A61K 6/06, A61L 24/02

(54) **COMPOSITION DE CIMENT À USAGE DENTAIRE**
ZEMENTZUSAMMENSETZUNG FÜR DENTALE ZWECKE
CEMENT COMPOSITION FOR DENTAL USE

(30) Priorité: 07.04.2010 FR 1052631
(43) Date de publication de la demande: 13.02.2013
(60) Demande divisionnaire: 16175381.9
(73) Titulaire: Septodont ou Septodont SAS ou Specialites Septodont, 94100 Saint Maur des Fossés (FR)
(72) Inventeur: RICHARD, Gilles, F-91560 Crosne (FR); MARIE, Olivier, F-91450 Soisy sur Seine (FR)
(74) Mandataire: Icosa
(86) Numéro de dépôt international: PCT/FR2011/050764
(87) Numéro de publication internationale: WO 2011/124841

(56) Documents cités:
- WO-A1-93/21122
- WO-A2-2004/017929
- WO-A2-2008/102214
- FR-A1- 2 603 274
- US-A1- 2002 045 678
- US-A1- 2003 127 026

## Description

La présente invention a pour objet une nouvelle composition, pour la restauration de substance minéralisée, en particulier dans le domaine dentaire. Plus spécifiquement, la composition de l'invention est utile pour la restauration dé couronnes dentaires ayant subi une perte de substance, pour le traitement endodontique, et en chirurgie dentaire.

Par restauration au sens de la présente invention, on entend la reconstitution de dents délabrées à la suite d'un choc ou d'une infection bactérienne ou virale, notamment une carie, en particulier l'obturation de cavités.

Dans la pratique dentaire, l'amalgame d'argent a été utilisé depuis longtemps pour la restauration de couronnes dentaires, et a été particulièrement apprécié car il permettait une durée d'obturation de 14 ans en moyenne. Toutefois, sa formulation, comprenant du mercure, posait des problèmes de sécurité sanitaire du patient en cas de relargage dans la salive. Par ailleurs, son aspect métallique était inesthétique.

Les résines composites qui ont été proposées ensuite en alternative à l'amalgame d'argent résolvaient le problème esthétique, mais ne permettait une obturation efficace que pendant environ 7 ans.

Les ciments verres ionomères qui ont également été développés proposaient une solution esthétiquement possible, mais présentent des inconvénients qui a limité généralement leur utilisation à l'obturation des dents temporaires : notamment, il est impossible de les placer en contact direct avec les tissus pulpaires et leur résistance mécanique est limitée.

Obtenir des propriétés mécaniques améliorées, notamment une bonne résistance mécanique à la compression, une durée de vie allongée, ainsi qu'une excellente biocompatibilité, est un véritable défi dans le domaine des compositions dentaires. En effet, les compositions dentaires de l'art antérieur, qui utilisent des ciments de type Portland, ont des bonnes propriétés biologiques mais des propriétés mécaniques relativement médiocres. La Demanderesse a analysé la composition des ciments de Portland, et a identifié que la présence d'aluminate nuisait aux propriétés mécaniques de la composition finale. Un problème technique à résoudre était donc d'être capable de produire une composition dentaire ne contenant pas d'aluminate.

**Composition du ciment Portland**

| | Formule chimique | Nom courant | Quantité en poids dans le ciment Portland (%w/w) |
|---|---|---|---|
| Tricalcium silicate | **Ca₃SiO₅** | C3S | 40-65 |
| Dicalcium silicate | **Ca₂SiO₄** | C2S | 10-20 |
| Tricalcium aluminate | **Ca₃Al₂O₆** | C3A | 10 |
| Tetracalcium aluminoferrite | **Ca₄Al₂Fe₂O₁₀** | C4AF | 10 |
| Calcium sulfate dihydrate | **CaSO₅ 2H₂O** | CSH2 | 2-5 |

Compositions des ciments de type Portland sont décrites par le document WO-A-93/21122.

La composition objet du brevet EP 1 531 779, déposé en 2003 par la Demanderesse, propose une préparation pour réaliser un matériau de restauration, qui permet l'obtention d'un matériau ayant à la fois une esthétique et une résistance à la compression acceptables, de l'ordre de 100 à 200 MPa.

Toutefois, il existe toujours une demande de la part des patients et des praticiens, pour des compositions dentaires ayant de bonnes propriétés mécaniques, et la présente invention se situe comme une alternative aux compositions objet du brevet EP 1 531 779.

Dans le cadre des recherches que la Demanderesse mène pour optimiser constamment les compositions dentaires qu'elle commercialise, la Demanderesse a réalisé, à l'encontre du préjugé de l'homme du métier, que les compositions ayant les meilleures propriétés mécaniques n'étaient pas celles faites d'empilements de gros cristaux de silicate calcique, dans lesquels s'inséraient de petits cristaux de carbonate de calcium, mais celles faites de cristaux de silicate calcique ayant le même ordre de grandeur de taille que les cristaux de carbonate de calcium. Le fait d'utiliser des cristaux de carbonate de calcium ayant une granulométrie d50 de l'ordre de 2 à 4 microns permettait en outre d'éviter des phénomènes de démixtion de la composition, conduisant à de nombreux rebuts industriels.

En partant de cette constatation, la Demanderesse a ensuite réussi à maitriser complètement le procédé de fabrication des compositions selon l'invention, et en particulier le processus de formation du composé faisant fonction de joint entre les différents cristaux dans la composition finale.

Ce composé ayant fonction de joint entre les différents cristaux présents dans la composition finale est un produit de la réaction du silicate calcique avec l'eau. Il est généralement nommé CSH. Par exemple, dans le cas du silicate tricalcique :

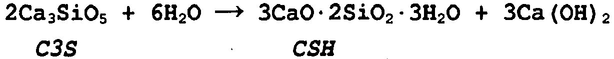

Le CSH, est un produit de formule générale mCaO.nSiO₂.pH₂O dans laquelle n et m, chacun indépendamment, varient de 1 à 3, et p varie de 3 à 6. Dans le mode de réalisation du silicate tricalcique, m est 3, n est 2 et p est 3. Le CSH est un hydrate qui se forme par germination hétérogène sur la surface des cristaux de silicate tricalcique et se développe par agrégation de nano-cristaux pour couvrir toute la surface des cristaux de silicate calcique. C'est cette agrégation qui, en multipliant les contacts entre les cristaux, est à l'origine de l'augmentation dans le temps de la résistance mécanique de la composition de l'invention.

Ainsi, l'invention a pour objet une composition dans laquelle la nature et la granulométrie des solides est parfaitement contrôlée, et la quantité de CSH formée est également maitrisée.

Plus précisément, la présente invention a pour objet une composition dentaire comprenant :
- de 5 à 65%, de préférence 8 à 60%, plus préférentiellement 10 à 35% en poids par rapport au poids total de la composition, de silicate calcique, ledit silicate calcique étant de préférence choisi dans le groupe comprenant le silicate tricalcique et/ou un mélange de silicate dicalcique et de silicate tricalcique,
- de 1 à 20%, de préférence de 2 à 15% en poids par rapport au poids total de la composition, de carbonate de calcium et
- de plus de 0 à 50%, de préférence 1 à 40% en poids par rapport au poids total de la composition, de mCaO·nSiO₂·pH₂O (CSH) dans laquelle m et n, chacun indépendamment, varient de 1 à 3 et p varie de 3 à 6 ; avantageusement, ledit pourcentage de CSH est de plus de 0 à moins de 3.5% en poids par rapport au poids total de la composition pendant 1 à 6 minutes à compter de la formation de ladite composition, cette période étant appelée temps de travail ; avantageusement, ledit pourcentage de CSH est de 3.5 à 8%, de préférence, 4 à 6 % en poids par rapport au poids total de la composition pendant 6 à 15 minutes à compter de la formation de la composition, cette période étant appelée temps de prise ;
   la granulométrie d50 des cristaux de silicate calcique étant de 1 à 5,5 µm;
   la granulométrie d50 des cristaux de carbonate de calcium étant de 1 à 5,5 µm; et
   le rapport entre la granulométrie d50 des cristaux de silicate calcique et la granulométrie d50 des cristaux de carbonate de calcium étant inférieur à 10.
- le rapport entre la granulométrie d50 des cristaux de silicate calcique et la granulométrie d50 des cristaux de carbonate de calcium est inférieur à 10 ; de préférence ce rapport est de 0,1 à 9, très préférentiellement de 0,2 à 5, encore plus préférentiellement de 0,5 à 2.

Selon l'invention, la granulométrie est.mesurée par un appareillage Beckman-Coulter Granulomètre LS230 avec module SVM. La valeur « d10 » signifie que 10% des cristaux du composé considéré ont un diamètre inférieur à ladite valeur. La valeur « d50 » signifie que 50% des cristaux du composé considéré ont un diamètre inférieur à ladite valeur. La valeur « d90 » signifie que 90% des cristaux du composé considéré ont un diamètre inférieur à ladite valeur. La valeur « d99,9 » signifie que 99,9% des cristaux du composé considéré ont un diamètre inférieur à ladite valeur.

La d50 du silicate calcique est de 1 à 5.5 microns, de préférence 3 à 4 microns. La d50 du carbonate de calcium est de 1 à 5,5 microns, de préférence 2 à 4 microns. La composition selon l'invention est telle que le rapport entre la granulométrie d50 des cristaux de silicate calcique et la granulométrie d50 des cristaux de carbonate de calcium est inférieure à 10 ; de préférence ce rapport est de 0,1 à 9, très préférentiellement de 0,2 à 5, encore plus préférentiellement de 0,5 à 2.

Avantageusement, la d10 du silicate calcique est de 0,2 à 1 microns, de préférence 0,7 à 0,8 micron. Avantageusement la d10 du carbonate de calcium est de 0,2 à 1 micron, de préférence 0,6 à 0,8 micron. Suivant un mode de réalisation de l'invention, la composition selon l'invention est telle que le rapport entre la granulométrie d10 des cristaux de silicate calcique et la granulométrie d10 des cristaux de carbonate de calcium est inférieure à 10 ; de préférence ce rapport est de 0,1 à 9, très préférentiellement de 0,2 à 4, encore plus préférentiellement de 0,5 à 2.

Avantageusement la d90 du silicate calcique est de 6 à 20 microns, de préférence de 7 à 14 microns. Avantageusement la d90 du carbonate de calcium est de 6 à 20 microns, de préférence 9 à 14 microns. Suivant un mode de réalisation de l'invention, la composition selon l'invention est telle que le rapport entre la granulométrie d90 des cristaux de silicate calcique et la granulométrie d90 des cristaux de carbonate de calcium est inférieure à 10 ; de préférence ce rapport est de 0,1 à 9, très préférentiellement de 0,2 à 5, encore plus préférentiellement de 0,5 à 4.

Avantageusement la d99,9 du silicate calcique est de 9 à 25 microns, de préférence de 10 à 15 microns. Avantageusement la d99,9 du carbonate de calcium est de 15 à 25 microns, de préférence 22 à 24 microns. Suivant un mode de réalisation de l'invention, la composition selon l'invention est telle que le rapport entre la granulométrie d99,9 des cristaux de silicate calcique et la granulométrie d99,9 des cristaux de carbonate de calcium est inférieur à 10 ; de préférence ce rapport est de 0,1 à 9, très préférentiellement de 0,2 à 5, encore plus préférentiellement de 0,5 à 4.

Suivant un premier mode de réalisation, le silicate calcique est du silicate tricalcique pur.

Suivant un autre mode de réalisation, le silicate calcique est un mélange comprenant ou comprenant essentiellement du silicate tricalcique et du silicate dicalcique. Avantageusement, ce mélange est tel qu'il comprend au plus 10% en poids de silicate dicalcique par rapport au poids total des silicates calciques présents dans la composition, typiquement le silicate dicalcique et le silicate tricalcique. Suivant un mode de réalisation préféré, le mélange comprend ou est constitué par 0 à 10% de silicate dicalcique et 90 à 100% de silicate tricalcique, en poids par rapport au poids total des silicates calciques. Le terme « est constitué par » signifie notamment que le mélange ne contient pas d'autre silicate calcique que le silicate dicalcique et le silicate tricalcique.

Suivant un mode de réalisation préféré, le mélange ne contient pas d'aluminate. Suivant un autre mode de réalisation préféré, le mélange ne contient pas de sulfate de calcium.

Selon l'invention la cinétique de prise est une fonction mathématique, de préférence une régression linéaire, de la résistance à la compression de la composition selon l'invention. Suivant un mode de réalisation préféré, le pourcentage de CSH présent dans la composition de l'invention peut être mesuré par régression linéaire de la résistance à la compression de la composition. Ainsi, une composition selon l'invention qui a été fabriquée depuis environ 15 minutes, a une résistance à la compression d'environ 43,5 MPa, correspondant à environ 5 % en poids de CSH dans la composition.

Avantageusement, la composition comprend un accélérateur de prise, de préférence de l'oxyde de calcium. L'oxyde de calcium peut être présent dans une quantité variant de 0 à 3%, de préférence 0,1 à 1% en poids par rapport au poids total de la composition. L'oxyde de calcium a pour fonction d'accélérer l'hydratation des cristaux de silicate calcique, et donc la formation de CSH et la prise.

Suivant un mode de réalisation préféré, la composition selon l'invention comprend au moins un radio-opacifiant, de préférence de l'oxyde de zirconium et l'oxyde de bismuth. Les radio-opacifiants augmentent la radio-opacité de la composition selon l'invention, permettant ainsi le contrôle radiographique de la restauration effectuée par le praticien. Avantageusement, la quantité de radio-opacifiant, de préférence d'oxyde de zirconium dans la composition selon l'invention est de 2 à 25%, de préférence de 5 à 20% en poids par rapport au poids total de la composition. Suivant un mode de réalisation préféré, le radio-opacifiant utilisé, de préférence l'oxyde de zirconium, a une granulométrie du même ordre de grandeur que le carbonate de calcium et le silicate calcique. Avantageusement, la d10 de l'oxyde de zirconium est de 0,1 à 0,4 microns, de préférence environ 0,2 microns. Avantageusement, la d50 de l'oxyde de zirconium est de 1 à 4 microns, de préférence de 2 à 3 microns. Avantageusement, la d90 de l'oxyde de zirconium est de 6 à 8 microns, de préférence environ 7 microns. Suivant un mode de réalisation préféré, le rapport entre la granulométrie d50 du silicate calcique et la granulométrie d50 du radio-opacifiant est inférieur à 10 ; de préférence ce rapport est de 0,1 à 9, très préférentiellement de 0,2 à 5, encore plus préférentiellement de 0,5 à 4.

Au sens de la présente invention, le terme « environ » suivi d'une donnée chiffrée signifie la donnée chiffrée, plus ou moins 10% de la donnée chiffrée.

Avantageusement, la composition selon l'invention comprend en outre des pigments, de préférence des oxydes de fer. Avantageusement, lesdits oxydes de fer sont choisis parmi les oxydes de fer jaune, rouge et brun. Avantageusement, la composition comprend moins de 1.5%, de préférence de 0,5 à 1% en poids de pigments par rapport au poids total de la composition.

Avantageusement, la composition comprend en outre un plastifiant, par exemple un Néomère®, du type notamment commercialisé par la société Chryso ; ou un Glénium^{®}, du type notamment commercialisé par la société BASF. Avantageusement, la composition selon l'invention comprend 0.1 à 1% en poids de plastifiant, par rapport au poids total de la composition.

Avantageusement, le module élastique de la composition de l'invention, dans la période de temps allant de 1 à 6 minutes à compter de la fin du mélange, est inférieur à 20 MPa, de préférence inférieur à 10 MPa, et dans la période de 11 à 20 minutes, excède 100 MPa. Le module élastique est utile pour mesurer le temps de travail de la composition de l'invention, qui correspond au temps entre la fin du mélange, et le début de la prise, et qui est de 1 à 6 minutes, de préférence d'environ 6 minutes, et pour mesurer le temps de prise, qui est de 6 à 15 minutes, de préférence d'environ 10 minutes.

Le module élastique est mesuré par un rhéomètre à déformation contrôlée du type ARES commercialisé par la société Rheometric Scientific, Inc, Piscataway, NJ, USA, dans les conditions décrites ci-après : après le mélange, la composition est mise en place entre deux plans parallèles striés de 6 mm de diamètre dont l'entrefer est de 2 mm. Seul le plan inférieur est à la température contrôlée de 37°C, une enceinte fermée maintenant l'ensemble de l'échantillon en température et à 100% d'humidité relative pour éviter son séchage. Les conditions expérimentales sont les suivantes : fréquences des oscillations à 1 radian par seconde, déformation appliquée : 0,0005%. Dans ces conditions, la déformation est inférieure à la déformation critique au delà de laquelle toute pâte de ciment se déstructure (de l'ordre de 0,0015%) et la contrainte transmise est proportionnelle à la déformation. On peut ainsi mesurer l'évolution du module élastique G' du matériau en fonction du temps, sans aucune modification de structure du matériau.

De préférence, la résistance à la compression de la composition de l'invention à une heure, est supérieure à 50 MPa et atteint, de l'ordre de 200 MPa après quelques jours, typiquement 7 jours, pour atteindre 300 MPa à un mois.

Selon un mode de réalisation, la résistance à la compression de la composition de l'invention à 24 heures, est supérieure à 50 MPa. Selon un autre mode de réalisation, la résistance à la compression de la composition de l'invention à 24 heures, est supérieure à 100 MPa. Selon un autre mode de réalisation, la résistance à la compression de la composition de l'invention à 24 heures, est supérieure à 150 MPa.

La résistance à la compression est mesurée en référence à la norme ISO 9917-1 :2007. Les éprouvettes sont préparées après le mélange, en utilisant des moules cylindriques en téflon de 4 mm de diamètre et de 6 mm de hauteur. Les moules sont remplis en prenant garde d'éliminer les bulles d'air de la pâte et stockés dans un incubateur pendant 15 minutes à 37°C et 100% d'humidité relative pendant le temps de prise désiré. Les éprouvettes sont ensuite démoulées et stockées sous eau distillé à 37°C pendant le temps de prise désiré. Les échantillons sont ainsi conservés dans des conditions proches de leurs futures conditions d'utilisation clinique. La résistance à la compression des éprouvettes est mesurée à quatre échéances, soit 1 heure, 1 jour, 7 jours et 28 jours à l'aide d'une presse Universal (Modèle 2/M MTS Systems 1400, Eden Prairie, Minneapolis, USA) avec une vitesse de déplacement de 0,5 mm par minute.

Avantageusement, la porosité de la composition selon l'invention est inférieure à 10%, de préférence comprise entre 2 et 8%, préférentiellement à environ 7%. La porosité de la composition de l'invention a été mesurée par porosimétrie à intrusion de mercure (PIM) à l'aide d'un instrument Micromeritics Autopore III dans un domaine de pression variant de 0,001 à 414 MPa, ce qui permet d'accéder à des pores d'un diamètre d'entrée compris entre 3nm et 360 microns. Alternativement, la mobilité des ions, qui indique le nombre de pores et la taille des pores, est mesurée par résistance électrique : après la prise initiale, le matériau continu à s'améliorer en termes de structure interne, pour devenir de plus en plus dense et de moins en moins poreux.

L'invention a également pour objet un procédé de préparation de la composition selon l'invention, dans lequel on effectue un mélange d'une phase solide et d'une phase aqueuse, par exemple à l'aide d'un malaxeur vibreur classique à 4200 oscillations par minutes pendant 10 à 50 secondes, typiquement 30 secondes.

La phase solide comprend du silicate tricalcique ou un mélange de silicate tricalcique et de silicate dicalcique. Suivant un premier mode de réalisation, la phase solide comprend des silicates calciques choisis parmi le silicate tricalcique et un mélange de silicate tricalcique Ca₃SiO₅ et de silicate dicalcique Ca₂SiO₄. Avantageusement, ledit mélange de silicate tricalcique et de silicate dicalcique comprend au plus 10% en poids de silicate dicalcique par rapport au poids total des silicates calciques présents dans la composition, typiquement le silicate dicalcique et le silicate tricalcique. Avantageusement, le silicate tricalcique utilisé dans la composition de l'invention est tel que sa teneur en chaux libre est inférieure à 2,0 % en poids par rapport au poids de silicate tricalcique. Suivant un mode de réalisation préféré, le ratio molaire calcium sur silice dans le silicate tricalcique de l'invention est de 2,95 à 3,05. Le silicate tricalcique, ou le mélange de silicate tricalcique et de silicate dicalcique, utilisés dans la composition initiale destinée à devenir la composition de l'invention, se présentent sous la forme d'une poudre fine, blanche ou sensiblement blanche, hygroscopique.

La phase solide comprend en outre du carbonate de calcium CaCO3 qui se présente sous la forme d'une poudre blanche ou sensiblement blanche pratiquement insoluble dans l'eau.

Suivant un premier mode de réalisation préféré, la phase solide comprend en outre un radio-opacifiant, du type notamment de l'oxyde de zirconium ZrO2 ou de bismuth, qui se présentent sous la forme d'une poudre blanche pratiquement insoluble dans l'eau.

Suivant un second mode de réalisation préféré, la phase solide comprend en outre de l'oxyde de calcium CaO qui se présente sous la forme d'une poudre granuleuse, blanche ou légèrement jaunâtre à grisâtre dans les acides dilués et pratiquement insoluble dans l'éthanol.

Avantageusement, la phase solide comprend des pigments, notamment des oxydes de fer. De préférence, la phase solide comprend de l'oxyde de fer jaune Fe(O)OH, contenant au minimum 60% de fer en poids par rapport au poids total de ce pigment, qui se présente sous la forme d'une poudre jaune pratiquement insoluble dans l'eau et dans l'alcool ; de l'oxyde de fer rouge Fe(O)OH, qui contenant au minimum 60% de fer en poids par rapport au poids total de ce pigment, qui se présente sous la forme d'une poudre rouge pratiquement insoluble dans l'eau et dans l'alcool ; de l'oxyde de fer brun, qui est un mélange de trois oxydes de fer : l'oxyde de fer rouge : Fe2O3, l'oxyde de fer jaune : Fe2O3, H2O, l'oxyde de fer noir : Fe3O4 ; l'oxyde de fer brun contient plus de 60% de fer en poids par rapport au poids total de ce pigment et se présente sous la forme d'une : poudre de couleur brune, magnétique et électrostatique, pratiquement insoluble dans l'eau et dans l'éthanol à 96 pour cent.

Suivant un mode de réalisation préféré, la phase solide comprend :
- un mélange de silicate tricalcique et de silicate dicalcique comprenant plus de 90% en poids de silicate tricalcique et moins de 10% en poids de silicate dicalcique, rapporté au poids total des silicates calciques
- du carbonate de calcium
- de l'oxyde de calcium,
- optionnellement, mais préférentiellement, un radio-opacifiant, très préférentiellement de l'oxyde de zirconium,
- et optionnellement des pigments, par exemple des oxydes de fer.

La phase aqueuse comprend du chlorure de calcium, de préférence du chlorure de calcium dihydraté CaCl2. 2H2O, un agent réducteur d'eau qui est du polycarboxylate, à savoir une base d'acide polyméthyl acrylique partiellement estérifié par des chaînes polyoxyde éthylénique, très préférentiellement du type Néomère^{®} ou Glénium^{®}, et de l'eau apte à la préparation de médicaments. Avant dissolution dans la phase aqueuse, le chlorure de calcium se présente sous forme de poudre et peut également être présent dans la phase solide.

La phase solide et la phase aqueuse sont mélangées dans un rapport massique phase solide sur phase aqueuse allant de 2 à 4,5, de préférence allant de 3 à 4. De préférence, on mélange 700 mg de phase solide avec 170 microlitres de phase aqueuse de densité supérieure à 1, d'environ 1,2.

La réaction entre le silicate calcique et l'eau se produit à la surface de chaque cristal de silicate calcique. Le CSH et l'hydroxyde de calcium Ca(OH)₂ précipitent à la surface des cristaux et entre les pores des cristaux. Les coeurs des cristaux de silicate calcique ne réagissent pas, et sont progressivement entourés, jusqu'à être enrobés, de CSH relativement imperméable à l'eau, ce qui va progressivement diminuer les possibilités de réactions ultérieures. Le durcissement de la pâte est dû à la formation progressive de plus en plus de CSH sur les cristaux.

Selon un mode de réalisation de l'invention, la composition est une composition dentaire destinée à être en contact prolongé (supérieur à 30 jours) avec des tissus dentaires. La composition selon l'invention est particulièrement destinée à la restauration des lésions carieuses des couronnes dentaires.

La composition selon l'invention peut être utilisée comme matériau d'obturation apical ou canalaire et d'apexification.

La composition selon l'invention est également particulièrement utile pour le coiffage pulpaire direct ou indirect, en particulier pour le recouvrement de la partie atteinte de la pulpe, car elle maintient la vitalité de la pulpe dentaire. En particulier, elle peut être utilisée pour le coiffage de la pulpe radiculaire dans les opérations de pulpotomie (ablation de la pulpe camérale suivie du coiffage de la pulpe radiculaire). La composition selon l'invention est également particulièrement bien adaptée pour la réparation et la régénération du complexe dentino-pulpaire endommagé.

La composition selon l'invention favorise la formation de dentine et contribue ainsi à la création d'une barrière de dentine protectrice de la pulpe.

La composition selon l'invention peut être utilisée comme un substitut de dentine.

La composition selon l'invention peut également être utilisée en chirurgie endodontique, notamment pour une obturation à-rétro.

La composition selon l'invention peut être aussi utilisée comme matériau de comblement, notamment de comblement osseux, par exemple en chirurgie dentaire ou maxillaire.

Dans certaines applications, la composition selon l'invention est un implant.

La composition selon l'invention présente en outre l'avantage d'être parfaitement biocompatible, en conformité avec la norme ISO 7405-2008 : elle n'est pas cytotoxique, elle n'est pas sensibilisante, ce qui signifie qu'elle ne crée pas d'oedème ou d'érythème, elle n'est pas mutagénique, et ne crée pas d'irritation cutanée ni d'irritation oculaire.

La composition selon l'invention induit en outre la formation de neo-tissu de dentine in situ.

### Exemples

La composition selon l'invention résulte du mélange de 170 microlitres d'une phase aqueuse ci-dessous et de 700 mg d'une phase solide ci-dessous, par exemple à l'aide d'un malaxeur vibreur du type Vibreur Linea Tac commercialisé par la société italienne Montegrosso d'Asti à 4200 oscillations par minutes pendant 1 à 50 secondes, typiquement 30 secondes.

La composition est malaxée par le praticien dès que le mélange est réalisé. Le matériau n'évolue pas pendant les 6 premières minutes, qui correspondent au temps de travail du praticien. Le praticien l'utilise afin de mettre en place le matériau et le modeler à sa convenance.

**Exemples de Phases liquides**

| Désignation des matières premières de la phase liquide | Quantité (g) pour 100 g | | | | | |
|---|---|---|---|---|---|---|
| | Composition # | | | | | |
| | a | b | c | d | e | f |
| Chlorure de calcium dihydraté | 29,4 | 15 | 30,4 | 30,4 | 25,2 | 29,6 |
| Néomère^{®} | 2 | 2,5 | 1,5 | | | 1 |
| Glénium^{®} | | | | 1,5 | 2 | 1 |
| Eau purifiée | 68,6 | 82,5 | 68,1 | 68,1 | 72,8 | 68,4 |

**Exemples de Phases solides**

| Désignation des matières premières de la phase solide | Quantité (mg) pour 100 mg | | | | | | | | | |
|---|---|---|---|---|---|---|---|---|---|---|
| | Composition # | | | | | | | | | |
| | 1 | 2 | 3 | 4 | 5 | 6 | 7 | 8 | 9 | 10 |
| Silicate tricalcique | 75 | 79,82 | 80,67 | 76,5 | 75,05 | 87 | 78,7 | 75,7 | 70,7 | 69,5 |
| Oxyde de zirconium | | 5 | 5 | 5 | 15 | | | 19 | 12 | 24 |
| Oxyde de bismuth | 5 | | | 5 | | 7,4 | 15 | | 12 | |
| Oxyde de calcium | 0,35 | 0,15 | 0,25 | 0,1 | 0,3 | 0,5 | 0,2 | 0,3 | 0,3 | 1 |
| Carbonate de calcium | 19,6 | 15 | 14 | 13,36 | 9,5 | 5 | 6,05 | 5 | 5 | 5,5 |
| Oxyde de fer jaune | 0,05 | | 0,07 | 0,04 | | 0,02 | 0,03 | | | |
| Oxyde de fer rouge | | 0,03 | 0,01 | | 0,08 | 0,02 | 0,02 | | | |
| Oxyde de fer brun | | | | | 0,07 | | | | | |

La granulométrie d50 des cristaux de silicate tricalcique utilisés est comprise entre 3 et 3,5 µm, de préférence égale à 3 µm et celle des cristaux de carbonate de calcium est comprise entre 2 et 4 µm, de préférence égale à 3 µm.

**Exemples de compositions de l'invention à environ 5 à 6 minutes après la fin du mélange.**

| | Compositions (phase solide + phase liquide) | | | | | | | | | |
|---|---|---|---|---|---|---|---|---|---|---|
| | **1 + a** | | **3** + a | | **5 + a** | | **9 + a** | | **10 + a** | |
| | (mg) | (%) | (mg) | (%) | (mg) | %) | (mg) | (%) | (mg) | (%) |
| Silicate tricalcique | 511,92 | 56,63 | 551,61 | 61,02 | 512,27 | 56,67 | 481 ,82 | 53,30 | 473,42 | 52,37 |
| Oxyde de zirconium | 0 | 0 | 35 | 3,87 | 105 | 11,62 | 84 | 9,29 | 168 | 18,58 |
| Oxyde de bismuth | 35 | 3,87 | 0 | 0 | 0 | 0 | 84 | 9,29 | 0 | 0 |
| Oxyde de calcium | 2,45 | 0,27 | 1,75 | 0,19 | 2,1 | 0,23 | 2,1 | 0,23 | 7 | 0,77 |
| Carbonate de calcium | 137,2 | 15,18 | 98 | 10,84 | 66,5 | 7,36 | 35 | 3,87 | 38,5 | 4,26 |
| Oxyde de fer jaune | 0,35 | 0,04 | 0,49 | 0,05 | 0 | 0 | 0 | 0 | 0 | 0 |
| Oxyde de fer rouge | 0 | 0 | 0,07 | 0,01 | 0,56 | 0,06 | 0 | 0 | 0 | 0 |
| Oxyde de fer brun | 0 | 0 | 0 | 0 | 0, 49 | 0,05 | 0 | 0 | 0 | 0 |
| CSH | 18,54 | 2,05 | 18,54 | 2,05 | 18,54 | 2,05 | 18,54 | 2,05 | 18,54 | 2,05 |
| Chlorure de calcium dihydraté | 59,976 | 6,63 | 59,976 | 6,63 | 59,976 | 6,63 | 59,976 | 6,63 | 59,976 | 6,63 |
| Neomère^{®} | 4,08 | 0,45 | 4,08 | 0,45 | 4,08 | 0,45 | 4,08 | 0,45 | 4,08 | 0,45 |
| Eau | 134,484 | 14,88 | 134,484 | 14,88 | 134,484 | 14,88 | 134,484 | 14,88 | 134,484 | 14,88 |

La résistance à la compression des compositions précédentes a été testée 24 heures après le mélange des phases solides et liquides. Les résultats sont les suivants :

| | | | | | |
|---|---|---|---|---|---|
| **Composition** (phase solide + phase liquide) | **1 + a** | **3 + a** | **5 + a** | **9 + a** | **10 + a** |
| Résistance à la compression (MPa), 24h après mélange | / | 250 | 215 | 186 | 245 |

Les compositions testées dans ces exemples présentent une résistance à la compression supérieure à 50 MPa lorsque la composition a été fabriquée depuis plus de 24h.

Les compositions testées dans ces exemples présentent une porosité inférieure à 10%.

## Revendications

1. Composition comprenant:
- de 5 à 65%, en poids par rapport au poids total de la composition, de cristaux de silicate calcique ;
- de 1 à 20%, en poids par rapport au poids total de la composition, de cristaux de carbonate de calcium ;
- de plus de 0 à 50%, en poids par rapport au poids total de la composition, d'un composé de formule générale mCaO·nSiO₂·pH₂O dans laquelle m et n, chacun indépendamment varie de 1 à 3 et p varie de 3 à 6 ;
la granulométrie d50 des cristaux de silicate calcique étant de 1 à 5,5 µm ;
la granulométrie d50 des cristaux de carbonate de calcium étant de 1 à 5,5 µm ; et
le rapport entre la granulométrie d50 des cristaux de silicate calcique et la granulométrie d50 des cristaux de carbonate de calcium étant inférieur à 10.

2. Composition selon la revendication 1, dans laquelle le silicate calcique est du silicate tricalcique pur.

3. Composition selon la revendication 1, dans laquelle le silicate calcique est un mélange de silicate tricalcique et de silicate dicalcique, ledit mélange étant tel qu'il contient au plus 10% en poids de silicate dicalcique par rapport au poids total des silicates calciques présents dans la composition.

4. Composition selon l'une quelconque des revendications 1 à 3, comprenant en outre un accélérateur de prise.

5. Composition selon la revendication 4, dans laquelle l'accélérateur de prise est l'oxyde de calcium.

6. Composition selon l'une quelconque des revendications 1 à 5, comprenant en outre un radio-opacifiant.

7. Composition selon l'une quelconque des revendications 1 à 6, comprenant en outre au moins un pigment.

8. Composition selon l'une quelconque des revendications 1 à 7, comprenant en outre au moins un plastifiant.

9. Composition selon l'une quelconque des revendications 1 à 8, dans laquelle la résistance à la compression de ladite. composition est supérieure à 50 MPa lorsque la composition a été fabriquée depuis plus d'une heure.

10. Composition selon l'une quelconque des revendications 1 à **8**, dans laquelle la résistance à la compression de ladite composition est supérieure à 50 MPa lorsque la composition a été fabriquée depuis plus de 24 heures.

11. Composition selon l'une quelconque des revendications 1 à 10, dans laquelle la porosité de ladite composition est inférieure à 10%.

12. Procédé de préparation de la composition selon l'une quelconque des revendications 5 à 11, **caractérisé en ce que** l'on effectue un mélange d'une phase solide constituée d'un mélange de poudres et d'une phase aqueuse, ladite phase solide comprenant :
- du silicate tricalcique pur ou un mélange, de silicate tricalcique et de silicate dicalcique comprenant plus de 90% en poids de silicate tricalcique et moins de 10% en poids de silicate dicalcique, rapporté au poids total des silicates calciques ;
- du carbonate de calcium ;
- de l'oxyde de calcium ;
- et optionnellement de l'oxyde de zirconium et/ou au moins un pigment ;
ladite phase aqueuse comprenant du chlorure de calcium, un polycarboxylate et de l'eau, suivant un rapport massique phase solide sur phase aqueuse allant de 2 à 4,5.

13. Composition selon l'une quelconque des revendications 1 à 11 pour son utilisation dans la restauration de couronnes dentaires ayant subi une perte de substance, pour le traitement endodontique, et en chirurgie dentaire.

## Patentansprüche

1. Zusammensetzung, umfassend:
- von 5 bis 65 Gew%, bezogen auf das Gesamtgewicht der Zusammensetzung, von Calciumsilikatkristallen;
- von 1 bis 20 Gew%, bezogen auf das Gesamtgewicht der Zusammensetzung, von Calciumcarbonatkristallen;
- mehr als 0 bis 50 Gew%, bezogen auf das Gesamtgewicht der Zusammensetzung, einer Verbindung mit der allgemeinen Formel mCaO·nSiO₂·pH₂O, wobei m und n jeweils unabhängig von 1 bis 3 variieren und p von 3 bis 6 variiert;
die Granulometrie d50 der Calciumsilikatkristalle 1 bis 5,5 µm beträgt;
die Granulometrie d50 der Calciumcarbonatkristalle 1 bis 5,5 µm beträgt; und das Verhältnis zwischen der Granulometrie d50 der Calciumsilikatkristalle und der Granulometrie d50 der Calciumcarbonatkristalle weniger als 10 beträgt.

2. Zusammensetzung nach Anspruch **1**, wobei das Calciumsilikat reines Tricalciumsilikat ist.

3. Zusammensetzung nach Anspruch **1**, wobei das Calciumsilikat ein Gemisch aus Tricalciumsilikat und Dicalciumsilikat ist, wobei das Gemisch derart ist, dass es höchstens 10 Gew% Dicalciumsilikat mit Bezug auf das Gesamtgewicht der Calciumsilikate enthält, die in der Zusammensetzung vorhanden sind.

4. Zusammensetzung nach einem der Ansprüche **1** bis **3**, umfassend außerdem einen Abbindebeschleuniger.

5. Zusammensetzung nach Anspruch **4**, wobei der Abbindebeschleuniger Calciumoxyd ist.

6. Zusammensetzung nach einem der Ansprüche **1** bis **5**, umfassend außerdem ein Röntgenkontrastmittel.

7. Zusammensetzung nach einem der Ansprüche **1** bis **6**, umfassend außerdem mindestens ein Pigment.

8. Zusammensetzung nach einem der Ansprüche **1** bis **7**, umfassend außerdem mindestens einen Weichmacher.

9. Zusammensetzung nach einem der Ansprüche **1** bis **8**, wobei der Widerstand gegen die Kompression der Zusammensetzung größer als 50 MPa ist, wenn die Zusammensetzung seit mehr als einer Stunde hergestellt wurde.

10. Zusammensetzung nach einem der Ansprüche **1** bis **8**, wobei der Widerstand gegen die Kompression der Zusammensetzung größer als 50 MPa ist, wenn die Zusammensetzung seit mehr als 24 Stunden hergestellt wurde.

11. Zusammensetzung nach einem der Ansprüche **1** bis **10**, wobei die Porosität der Zusammensetzung geringer als 10 % ist.

12. Verfahren zur Herstellung der Zusammensetzung nach einem der Ansprüche **5** bis **11**, **dadurch gekennzeichnet, dass** die Mischung einer festen Phase, bestehend aus einem Gemisch von Pulvern, und einer wässrigen Phase durchgeführt wird, wobei die feste Phase Folgendes umfasst:
- ein reines Tricalciumsilikat oder ein Gemisch aus Tricalciumsilikat und Dicalciumsilikat, umfassend mehr als 90 Gew% Tricalciumsilikat und weniger als 10 Gew% Dicalciumsilikat bezogen auf das Gesamtgewicht der Calciumsilikate;
- Calciumcarbonat,
- Calciumoxyd;
- und optional Zirconiumoxyd und/oder mindestens ein Pigment;
wobei die wässrige Phase ein Calciumchlorid, ein Polycarboxylat und Wasser umfasst, gemäß einem Massenverhältnis von Festphase zu wässriger Phase, das von 2 bis 4,5 reicht.

13. Zusammensetzung nach einem der Ansprüche **1** bis **11** für ihre Verwendung bei der Wiederherstellung von Zahnkronen, die Substanz verloren haben, zur endodontischen Behandlung und in der Zahnchirurgie.

## Claims

1. A composition comprising:
- from 5% to 65%, by weight with respect to the total weight of the composition, of calcium silicate crystals;
- from 1% to 20%, by weight with respect to the total weight of the composition, of calcium carbonate crystals;
- from more than 0% to 50% by weight with respect to the total weight of the composition, of a compound of general formula mCaO.nSiO₂.pH₂O in which m and n each independently vary from 1 to 3 and p varies from 3 to 6;
wherein
the d50 granulometry of the calcium silicate crystals ranges from 1 to 5,5 µm;
the d50 granulometry of the calcium carbonate crystals ranges from 1 to 5,5 µm; and
the ratio between d50 granulometry of the calcium silicate crystals and d50 granulometry of the calcium carbonate crystals is less than 10.

2. The composition according to claim **1**, wherein the calcium silicate is pure tricalcium silicate.

3. The composition according to claim **1**, wherein the calcium silicate is a mixture of tricalcium silicate and dicalcium silicate, said mixture being such that it contains no more than 10% by weight of dicalcium silicate with respect to the total weight of the calcium silicates present in the composition.

4. The composition according to anyone of claims **1** to **3**, further comprising a setting accelerator.

5. The composition according to claim **4**, wherein the setting accelerator is calcium oxide.

6. The composition according to anyone of claims **1** to **5**, further comprising a radiopacifier.

7. The composition according to anyone of claims **1** to **6**, further comprising at least one pigment.

8. The composition according to anyone of claims **1** to **7**, further comprising at least one plasticiser.

9. The composition according to anyone of claims **1** to **8**, wherein the compressive strength of said composition is greater than 50 MPa when the composition was manufactured more than one hour ago.

10. The composition according to anyone of claims **1** to **8**, wherein the compressive strength of said composition is greater than 50 MPa when the composition was manufactured more than 24 hours ago.

11. The composition according to anyone of claims **1** to **10**, wherein the porosity of said composition is less than 10%.

12. A method of preparing the composition according to anyone of claims **5** to **11**, comprising mixing a solid phase consisting of a mixture of powders and an aqueous phase, said solid phase comprising:
- pure tricalcium silicate or a mixture of tricalcium silicate and dicalcium silicate comprising more than 90% by weight of tricalcium silicate and less than 10% by weight of dicalcium silicate, compared with the total weight of the calcium silicates;
- calcium carbonate;
- calcium oxide; and
- optionally, zirconium oxide and/or at least one pigment;
said aqueous phase comprising calcium chloride, a polycarboxylate and water, in mass ratio of solid phase to aqueous phase ranging from 2 to 4.5.

13. A composition according to anyone of claims **1** to **11**, for use in the restoration of a dental crown that has lost substance, for endodontic treatment, and in dental surgery.
